(19) Europäisches Patentamt
European Patent Office
Office européen des brevets

(11) Publication number: 0 597 303 A2

# (12) EUROPEAN PATENT APPLICATION

(21) Application number: 93117216.7

(22) Date of filing: 23.10.93

(51) Int. Cl.5: C07D 501/46, C07D 501/40, A61K 31/545

(30) Priority: 12.11.92 US 976250
12.11.92 US 976248

(43) Date of publication of application:
18.05.94 Bulletin 94/20

(84) Designated Contracting States:
AT BE CH DE DK ES FR GB GR IE IT LI LU MC NL PT SE

(71) Applicant: F. HOFFMANN-LA ROCHE AG
Postfach 3255
CH-4002 Basel(CH)

(72) Inventor: Dax, Scott Louis
1776 Richmond Road
Staten Island, N.Y. 10306(US)
Inventor: Keith, Dennis Dalton
8 Mendl Terrace
Montclair, N.J. 07042(US)
Inventor: Rossman, Pamela Loreen
82 Oakley Terrace
Nutley, N.J. 07110(US)
Inventor: Wei, Chung-Chen
244 Malapardis Road
Cedar Knolls, N.J. 07927(US)

(74) Representative: Martin, Björn et al
Grenzacherstrasse 124
Postfach 3255
CH-4002 Basel (CH)

## (54) Cephalosporin derivatives.

(57) Cephalosporin derivatives of the general formula

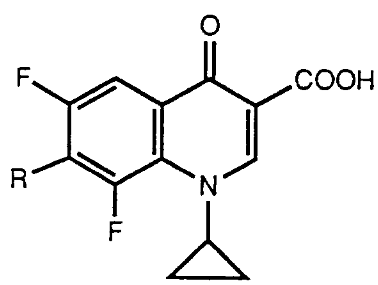

I

in which R represents one of the groups

EP 0 597 303 A2

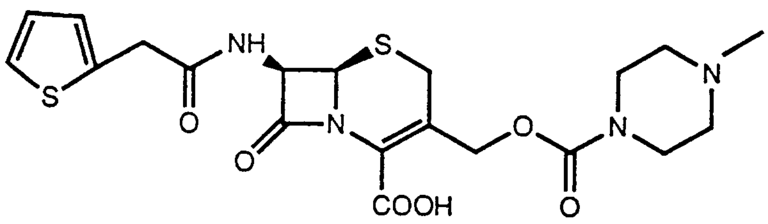

(a) and

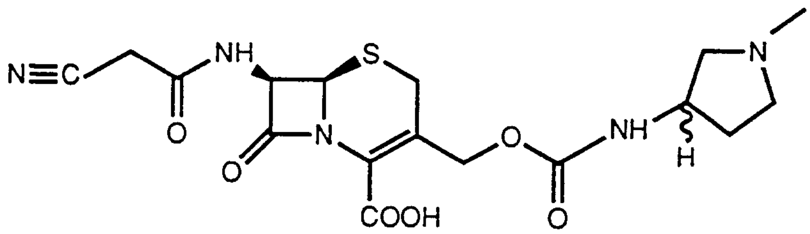

(b)

and the corresponding readily hydrolyzable, pharmaceutically acceptable esters and the pharmaceutically acceptable salts and hydrates of these compounds as well as a process for their manufacture and pharmaceutical preparations which contain these compounds, furthermore the use of the compounds in the control of illnesses and for the manufacture of the aforementioned preparations.

The present invention relates to cephalosporin derivatives substituted by a specifically substituted quinolonyl group, more particularly by a 3-carboxy-1-cyclopropyl-6,8-difluoro-1,4-dihydro-4-oxo-7-quinolonyl group. In particular, the invention relates to cephalosporin derivatives of the general formula

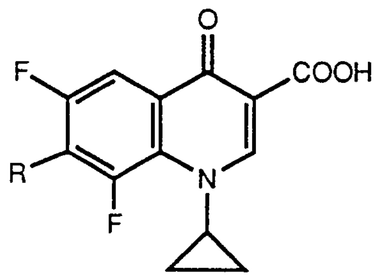

I

in which R represents one of the groups

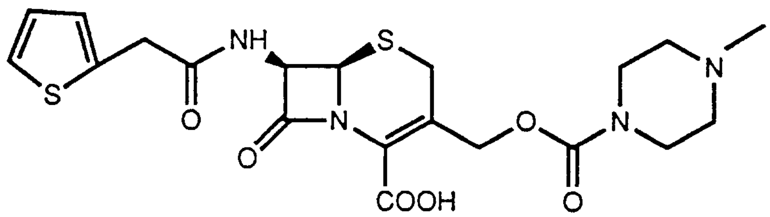

(a) and

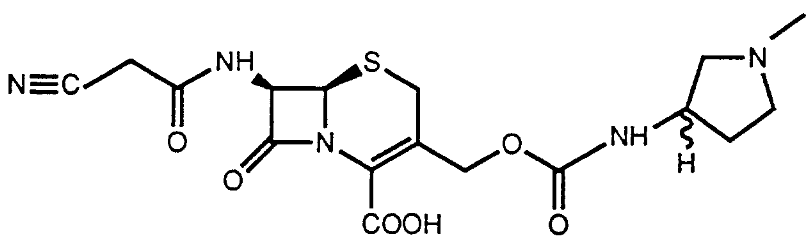

(b)

and the corresponding readily hydrolyzable, pharmaceutically acceptable esters and the pharmaceutically acceptable salts of these compounds and hydrates of compounds of formula I and of their esters and salts.

A specific embodiment of the compounds of the present invention concerns the compound of the formula

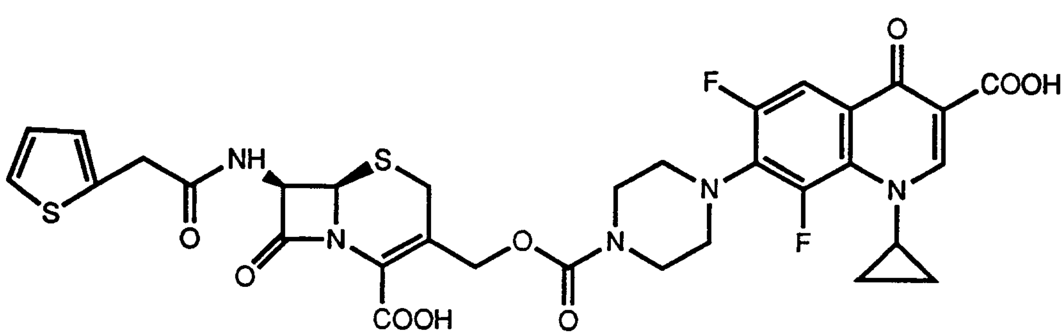

Ia

and the corresponding readily hydrolyzable, pharmaceutically acceptable esters and the pharmaceutically acceptable salts of this compound and hydrates of the compound of formula Ia and of its esters and salts.

Yet another embodiment of the compounds of the present invention concerns the compounds of the formula

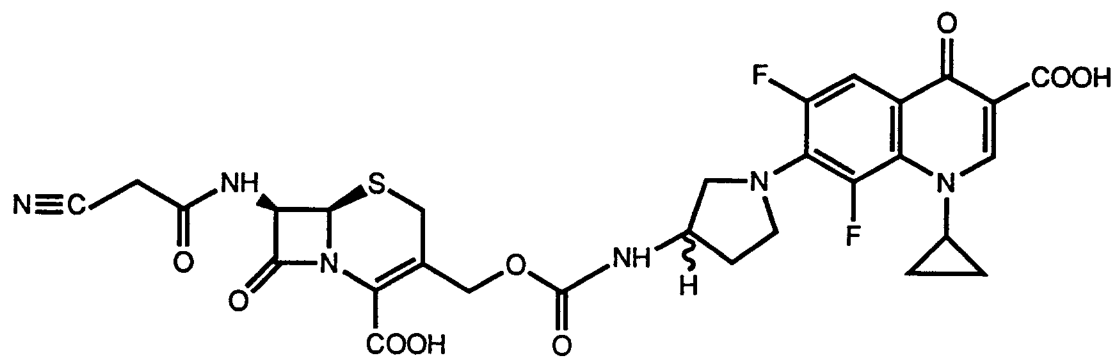

Ib

and the corresponding readily hydrolyzable, pharmaceutically acceptable esters and the pharmaceutically acceptable salts of this compound and hydrates of the compound of formula Ib and of its esters and salts.

Of interest is the S epimer

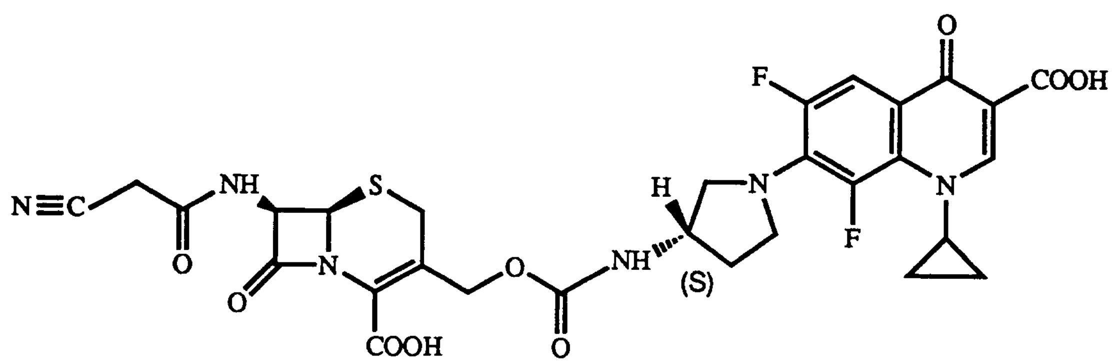

Iba

and the R epimer

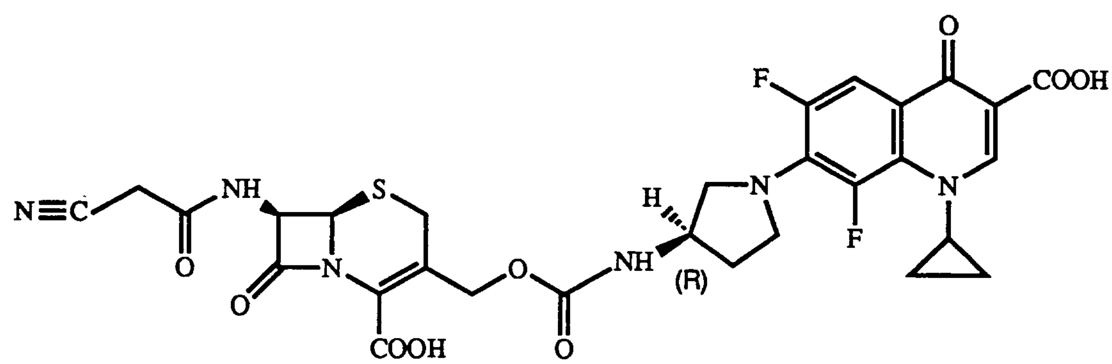

Ibb

and their corresponding readily hydrolyzable, pharmaceutically acceptable esters and the pharmaceutically acceptable salts of these compounds and hydrates of the compounds of formulas Iba and Ibb and of their esters and salt. The S epimer is most preferred.

The above compounds, their pharmaceutically acceptable salts and esters and hydrates of those compounds can be used as agents to combat bacterial infections (including urinary tract infections and respiratory infections) in mammalian species, for example, dogs, cats, horses, etc., and humans. These compounds exhibit activity against a broad range of both Gram-negative and Gram-positive bacteria.

The in vitro activity of the compounds of formula I of the present invention as measured by the Minimum Inhibitory Concentration (MIC) in micrograms/ml utilizing the Agar (Mueller Hinton) Dilution Method against a variety of Gram-positive and Gram-negative organisms, is set forth in Table 1.

Table 1: In Vitro Activity of Compounds of Formula I: MIC (μg/ml)

| Organism | Formula Ia | Formula Ib | Formula Iba | Formula Ibb |
|---|---|---|---|---|
| **Gram Negative** | | | | |
| Escherichia coli 257 | 0.0625 | 0.125 | 0.0313 | 0.125 |
| E. coli TEM-1a | 0.0625 | 0.125 | 0.0313 | 0.125 |
| E. coli ATCC 25922 | 0.0625 | 0.0625 | 0.0313 | 0.0625 |
| E. coli 4 | 0.125 | 0.125 | 0.0313 | 0.125 |
| E. coli B | 0.0313 | 0.0625 | ≤0.0156 | 0.0313 |
| E. coli 94 | 0.0625 | 0.125 | 0.0313 | 0.125 |
| E. coli Leo HA2 | ≤0.0156 | 0.0156 | ≤0.0156 | ≤0.0156 |
| E. coli Leo HA2M4 | ≤0.0156 | 0.0313 | ≤0.0156 | 0.0313 |
| E. coli 1269B | 0.0313 | 0.0625 | ≤0.0156 | 0.0625 |
| E. coli 1527E | ≤0.0156 | 0.0625 | ≤0.0156 | 0.0625 |
| E. coli 1573E | 0.0625 | 0.0625 | ≤0.0156 | 0.0625 |
| E. coli 1894 E | 0.0625 | 0.0625 | ≤0.0156 | 0.0625 |
| E. coli 2008 E | 0.0313 | 0.0625 | 0.0313 | 0.0625 |
| E. coli HMS1 | ≤0.0156 | 0.0313 | ≤0.0156 | 0.0313 |
| E. coli 2721B | 0.0313 | 0.0625 | ≤0.0156 | 0.0313 |
| E. coli 2722B | ≤0.0156 | ≤0.0156 | ≤0.0156 | ≤0.0156 |
| E. coli DC-0 | 2 | 1 | 0.5 | 2 |
| E. coli DC-2 | 0.25 | 0.25 | 0.0625 | 0.25 |
| Citrobacter freundii BS-16[a] | 0.0313 | 0.0625 | ≤0.0156 | 0.0313 |
| Cit. freundii EL-1408 | 0.25 | 0.25 | 0.125 | 0.5 |
| Klebsiella pneumoniae A | 0.25 | 0.25 | 0.0625 | 0.25 |
| K. pneumoniae 369 | 0.5 | 0.25 | 0.125 | 0.5 |
| K. oxytoca 1082EN | 0.0625 | 0.0625 | ≤0.0156 | 0.0625 |
| Enterobacter cloacae P99[a] | ≤0.0156 | 0.0313 | ≤0.0156 | ≤0.0156 |
| Enter. cloacae 4946 | 0.0625 | 0.125 | 0.0313 | 0.125 |
| Enter. cloacae 4192-2 | 0.0625 | 0.0625 | ≤0.0156 | 0.0313 |
| Enter. cloacae 5699 | 0.125 | 0.25 | 0.0625 | 0.25 |
| Serratia marcescens SM[a] | 0.5 | 0.25 | 0.125 | 0.25 |
| Ser. sp. 101 | 0.5 | 0.25 | 0.0625 | 0.125 |
| Ser. marcescens 1071 | 0.125 | 0.25 | 0.0313 | 0.125 |
| Ser. marcescens MA | 16 | 4 | 4 | 8 |
| Proteus vulgaris ATCC 6380[a] | 0.125 | 0.25 | 0.0625 | 0.125 |
| Prot. mirabilis 90 | 0.5 | 1 | 0.125 | 0.5 |
| Prot. vulgaris 1028 BC | 0.0625 | 0.125 | ≤0.0156 | 0.0313 |

| | | | | |
|---|---|---|---|---|
| Prot. vulgaris 100 | 0.125 | 0.25 | 0.0625 | 0.0625 |
| Prot. mirabilis 503-1136 | 0.5 | 0.25 | 0.0625 | 0.125 |
| Prot. mirabilis 620A | 0.0625 | 0.25 | 0.0625 | 0.125 |
| Prot. mirabilis 2 | 0.25 | 0.25 | 0.0625 | 0.125 |
| Prot. mirabilis 190 | 0.125 | 0.25 | 0.0313 | 0.0625 |
| Prov. rettgeri ATCC 9250 | 0.25 | 0.25 | 0.0625 | 0.25 |
| A. calcoaceticus PCI-3 | 0.25 | 0.25 | 0.0625 | 0.5 |
| Pseudomonas aeruginosa 5712 | 2 | 2 | 2 | 4 |
| Ps. aeruginosa 18SH[a] | 1 | 0.5 | 0.25 | 1 |
| Ps. aeruginosa ATCC 27853 | 1 | 1 | 1 | 2 |
| Ps. aeruginosa 8780 | N.D. | 1 | 0.5 | 2 |
| Ps. aeruginosa 765 | 2 | 1 | 0.5 | 2 |
| Ps. aeruginosa 1937E | 1 | 0.5 | 0.25 | 1 |
| Ps. aeruginosa 1973 E | 1 | 0.5 | 0.25 | 1 |
| Ps. aeruginosa K799/WT | 2 | 0.5 | 0.5 | 1 |
| Ps. aeruginosa K799/61 | 2 | 0.125 | 0.0625 | 0.25 |
| Ps. aeruginosa B | 0.5 | 0.25 | 0.25 | 1 |
| Ps. aeruginosa 56 | 1 | 0.5 | 0.25 | 1 |
| Ps. aeruginosa 56M | 0.25 | 0.25 | 0.0625 | 0.25 |
| Ps. aeruginosa 56-1B | 0.25 | 0.5 | 0.125 | 0.5 |
| Ps. cepacia 1973 B | 4 | 2 | 4 | 4 |
| Xan. maltophilia 2897B | 1 | 0.25 | 0.25 | 0.5 |
| **Gram Positive and Others** | | | | |
| Staphylococcus aureus Smith | 0.25 | 0.25 | 0.0625 | 0.125 |
| Staph. aureus 67[b] | 1 | 0.25 | 0.125 | 0.5 |
| Staph. aureus 753[b] | 0.5 | 0.25 | 0.0625 | 0.125 |
| Staph. pyogenes 4 | ≤0.0156 | 0.0625 | 0.0313 | 0.0625 |
| Staph. aureus ATCC 29213 | 0.5 | 0.25 | 0.0625 | 0.125 |
| Staph. aureus ATCC 25923 | 0.25 | 0.25 | 0.0625 | 0.25 |
| Staph. aureus 82 | 0.125 | 0.125 | 0.0625 | 0.125 |
| Staph. aureus 1059B | 0.25 | 0.25 | 0.0313 | 0.125 |
| Staph. aureus 3037B | 1 | 0.25 | 0.0625 | 0.125 |
| Staph. aureus Evans | 0.5 | 0.125 | 0.0313 | 0.0625 |
| Staph. aureus 3647B | 0.5 | 0.25 | 0.0313 | 0.125 |
| Staph. aureus 95 | 0.5 | 0.25 | 0.0313 | 0.125 |
| Staph. aureus 404 | 0.5 | 0.25 | 0.0313 | 0.125 |
| Micrococcus luteus PCI | ≤0.0156 | 0.125 | 0.0625 | 0.0625 |

| | | | | |
|---|---|---|---|---|
| Bacillus megaterium 164 | 0.125 | 0.0625 | 0.0313 | 0.0625 |
| Bacillus subtilis 558 | 0.0313 | 0.125 | 0.0313 | 0.0625 |
| Streptococcus pneumoniae 6303 | | 0.125 | 0.0313 | 0.0625 |
| Str. pneumoniae N82-012-MC | 0.0313 | 0.5 | 0.125 | 0.25 |
| Str. pneumoniae 6302 | | N.D. | 0.0625 | 0.0625 |
| Str. pyogenes 503-782 | ≤0.0156 | 0.0625 | 0.0313 | 0.0625 |
| Ent. faecalis ATCC 29212 | 4 | 2 | 0.5 | 2 |
| Ent. hirae ATCC 8043 | 2 | 2 | 0.5 | 1 |
| Mycobacterium phlei 78 | 0.125 | 0.25 | 0.0625 | 0.125 |
| Candida albicans 155 | >128 | >128 | >128 | >128 |

a = constitutive ß-lactamase producer; b = methicillin-resistant; N.D.: not determined.

For combating bacterial infections in mammals, both human and non-human, the compound of this invention can be administered to a mammal in need of such treatment in an amount of about 5 mg/kg/day to about 500 mg/kg/day, preferably about 10 mg/kg/day to 100 mg/kg/day, most preferably about 10 mg/kg/day to about 55 mg/kg/day. Those of ordinary skill in the art will appreciate the appropriate therapeutically effective amount necessary for combating the bacterial infections in the human and non-human mammal.

Modes of administration which have been used in the past to deliver penicillins and cephalosporins to the site of the infection are also contemplated for use with the compound of the present invention to mammalian hosts, both human and non-human. By way of illustration, such methods of administration include parenteral, e.g., intravenous or intramuscular, and enteral, e.g., as a suppository.

Appropriate therapeutically inert carriers useful for the different modes of administration can also be included, as well as other pharmaceutical adjuvants which are well known in the art.

Suitable carrier materials for intramuscular or intravenous injection solution unit dosage forms are, for example, water, alcohols, polyols, glycerol, and vegetable oils. Suitable carriers for suppositories are, for example, natural or hardened oils, waxes, fats, and semi-liquid or liquid polyols.

The usual stabilizing, preserving, wetting, emulsifying agents, consistency-improving agents, salts for varying the osmotic pressure, buffer substances, solubilizers, and antioxidants which are well known in the art also come into consideration as pharmaceutical adjuvants.

As readily hydrolyzable esters of the compound of formula I there are to be understood the compound of formula I, the carboxy group(s) of which (i.e., the 2-carboxy group) is/are present in the form of readily hydrolyzable ester groups. Examples of such esters, which can be of the conventional type, are the lower alkanoyloxyalkyl esters (e.g., the acetoxymethyl, pivaloyloxymethyl, 1-acetoxyethyl and 1-pivaloyloxyethyl ester), the lower alkoxycarbonyloxyalkyl esters (e.g., the methoxycarbonyloxymethyl, 1-ethoxycarbonyloxyethyl and 1-isopropoxycarbonyloxyethyl ester), the lactonyl esters (e.g., the phthalidyl and thiophthalidyl ester), the lower alkoxymethyl esters (e.g., the methoxymethyl ester) and the lower alkanoylaminomethyl esters (e.g., the acetamidomethyl ester). Other esters (e.g., the benzyl and cyanomethyl esters) can also be used. As used herein, alkyl, alkoxy, alkanoyl, and alkanoyloxy have their traditional meanings which are well known to those of ordinary skill in the art, and examples of which are listed above. "Lower" refers to those carbon-containing moieties having 1 to 6 carbon atoms.

Examples of salts of the compound of formula I are alkali metal salts such as the sodium and potassium salt, the ammonium salt, alkaline earth metal salts such as the calcium salt, salts with organic bases such as salts with amines (e.g., salts with N-ethylpiperidine, procaine, dibenzylamine, N,N'-dibenzylethylenediamine, alkylamines or dialkylamines) as well as salts with amino acids such as, for example, salts with arginine or lysine.

The compound of formula I as well as its salts and readily hydrolyzable esters can be hydrated. The hydration can be effected in the course of the manufacturing process or can occur gradually as a result of hygroscopic properties of an initially anhydrous product.

The products in accordance with the invention can be manufactured by

a) acylating a compound of the general formula

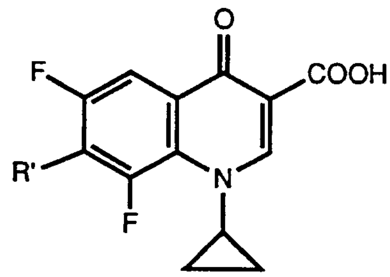

II

in which R' is a group corresponding to R carrying an unsubstituted amino group in position 7 of the cephalosporin nucleus,

or an acid addition salt thereof, or

b) for the manufacture of a readily hydrolyzable, pharmaceutically acceptable ester of a compound of formula I subjecting a carboxylic acid of formula I to a corresponding esterification, or

c) for the manufacture of pharmaceutically acceptable salts and hydrates of a compound of formula I and of hydrates of said salts converting a compound of formula I into a pharmaceutically acceptable salt or hydrate or into a hydrate of this salt.

The acylation in accordance with process variant a) of the process in accordance with the invention can be carried out according to methods which are known per se and which will be familiar to any person skilled in the art. For example, the compound of formula II can be acylated with the corresponding free carboxylic acid or a salt thereof with a base, whereby in this case the acylation is carried out in the presence of a suitable condensation agent and the compound of formula II is converted previously into a readily hydrolyzable ester. Suitable condensation agents are, for example, N,N'-disubstituted carbodiimides such as N,N'-dicyclohexylcarbodiimide, which are preferably used together with N-hydroxybenzotriasole or N-hydroxysuccinimide, 2-halopyridinium salts such as 2-chloro-1-methylpyridinium chloride, phosphorus oxychloride, thionyl chloride and oxalyl chloride.

It is, however, also possible to use the acylating carboxylic acid in the form of a reactive derivative. As reactive derivatives there come into consideration especially acid chlorides, acid anhydrides, mixed anhydrides (for example anhydrides with trifluoroacetic acid, bensenesulphonic acid, mesitylenesulphonic acid, p-toluenesulphonic acid and p-chlorosulphonic acid) and active thiol esters, for example S-(2-benzothiazolyl) thioesters. When this S-(2-benzothiazolyl) thioester is used, the amine of formula II is preferably employed in the form of an acid addition salt, e.g. a toluenesulphonate, methanesulphonate or hydrochloride.

The acylation is preferably carried out in the presence of a solubilizer for the amine of formula II such as N-methyl-N-(trimethylsilyl)trifluoroacetamide, triethylamine,pyridine or N-methylmorpholine. If desired, the acylation can be carried out in the presence of an acid-binding agent, with sodium hydrogen carbonate, potassium carbonate, triethylamine, pyridine or N-methylmorpholine especially coming into consideration as the acid-binding agent. Suitable solvents are, for example, cyclic ethers such as tetrahydrofuran and dioxan, halogenated lower hydrocarbons such as chloroform and methylene chloride, dimethylformamide, dimethylacetamide, acetonitrile, acetone and water as well as mixtures thereof. The reaction temperature can vary in a wide range and as a rule lies between -50 ° C and 50 ° C, preferably between about -10 ° C and 30 ° C.

For the manufacture of the readily hydrolyzable esters of the carboxylic acids of formula I in accordance with variant b), the carboxylic acid is preferably reacted with the corresponding halide, preferably with the iodide, which contains the ester group. The reaction can be expedited with the aid of a base, e.g. an alkali metal hydroxide, alkali metal hydrogen carbonate or alkali metal carbonate or an organic amine such as triethylamine, 1,8-diazabicyclo[5.4.0]undec-7-ene (DBU) or tetramethylguanidine. This reaction is preferably carried out in an inert organic solvent such as dimethylacetamide, hexamethylphosphoric acid triamide, dimethyl sulphoxide or, preferably, dimethylformamide. The temperature preferably lies in the range of about 0 to 40 ° C.

The manufacture of the salts and hydrates of the compounds of formula I and, respectively, of the hydrates of these salts in accordance with variant c) can be effected in a manner known per se, e.g. by reacting the carboxylic acid of formula I or salt thereof with an equivalent amount of the desired base,

conveniently in a solvent such as water or in an organic solvent such as ethanol, methanol, acetone or the like. Correspondingly, salt formation is achieved by the addition of an organic or inorganic acid. The temperature of the salt formation is not critical. In general, it lies at room temperature, but can also be slightly thereover or thereunder, for example in the range of 0°C to +50°C.

The manufacture of the hydrates is effected for the most part automatically in the course of the manufacturing process or as a consequence of hygroscopic properties of an initially anhydrous product. For the planned manufacture of a hydrate, a wholly or partially anhydrous product (carboxylic acid of formula I or ester or salt thereof) can be exposed to a moist atmosphere, e.g. at about +10°C to +40°C.

The following Reaction Schemes I-IV illustrate the process for producing the compounds of formula I.

## REACTION SCHEME I

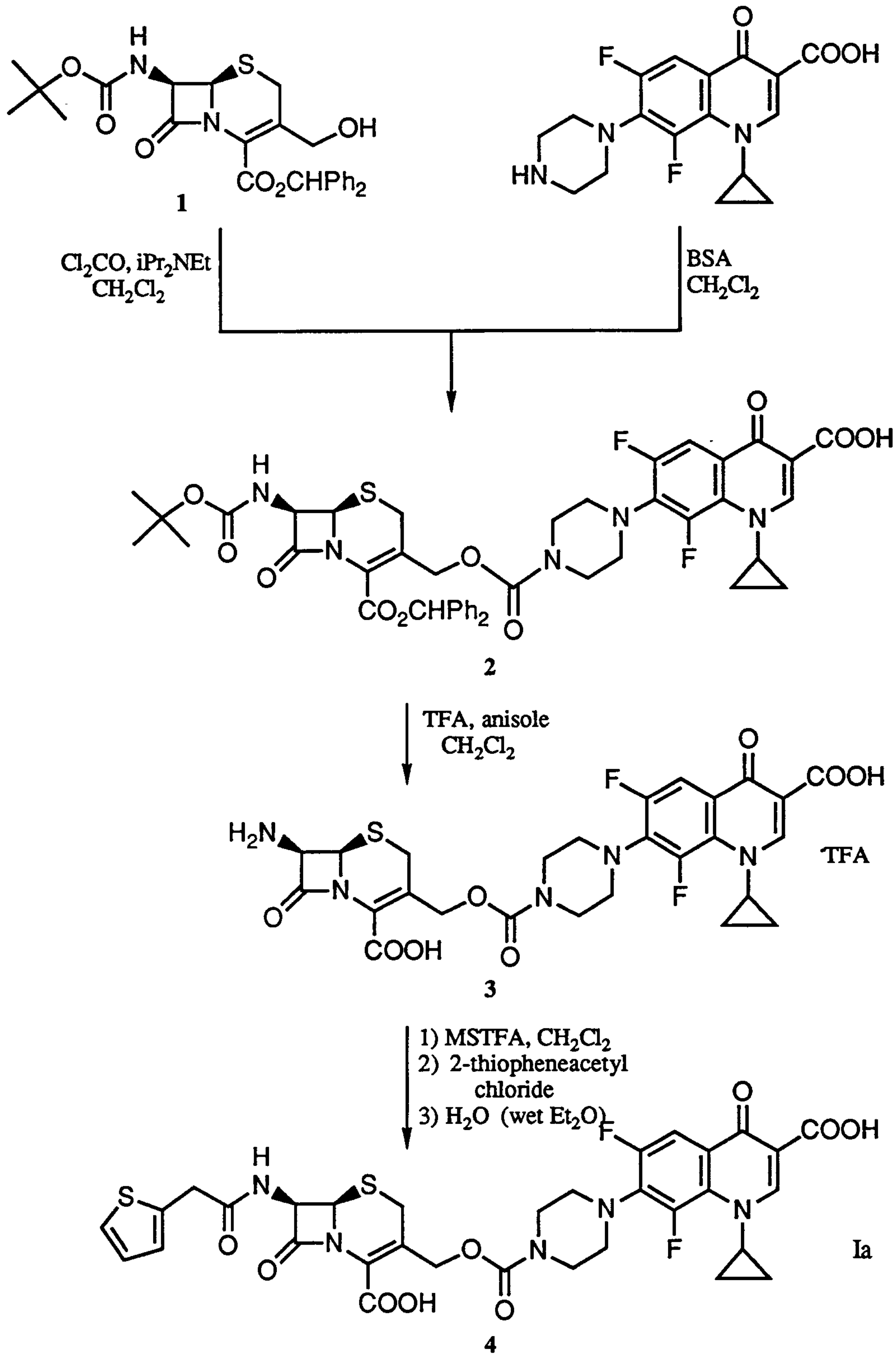

## REACTION SCHEME II

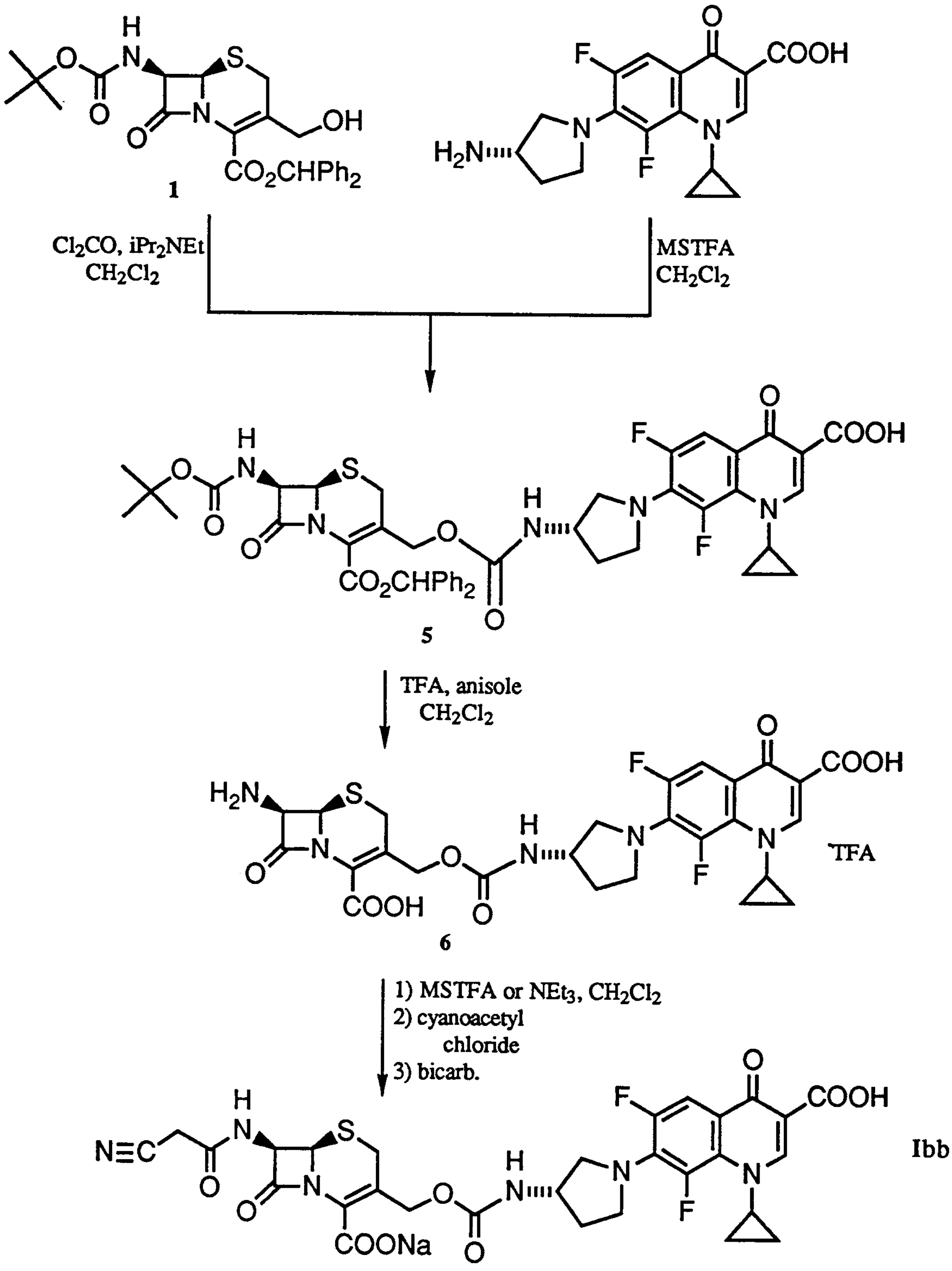


1

5

6

7

Ibb

## REACTION SCHEME III

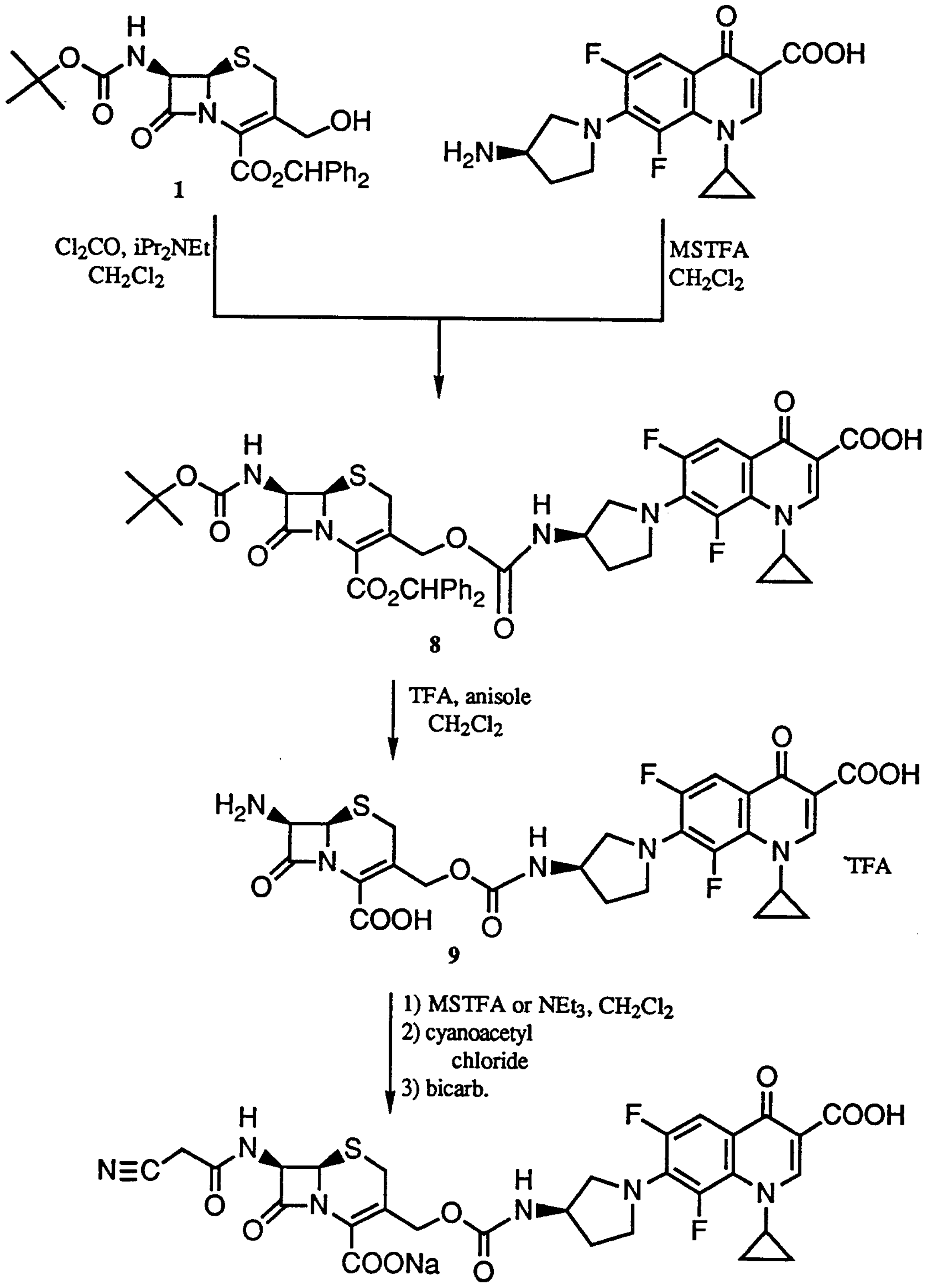


1

8

9

Iba

10

## REACTION SCHEME IV

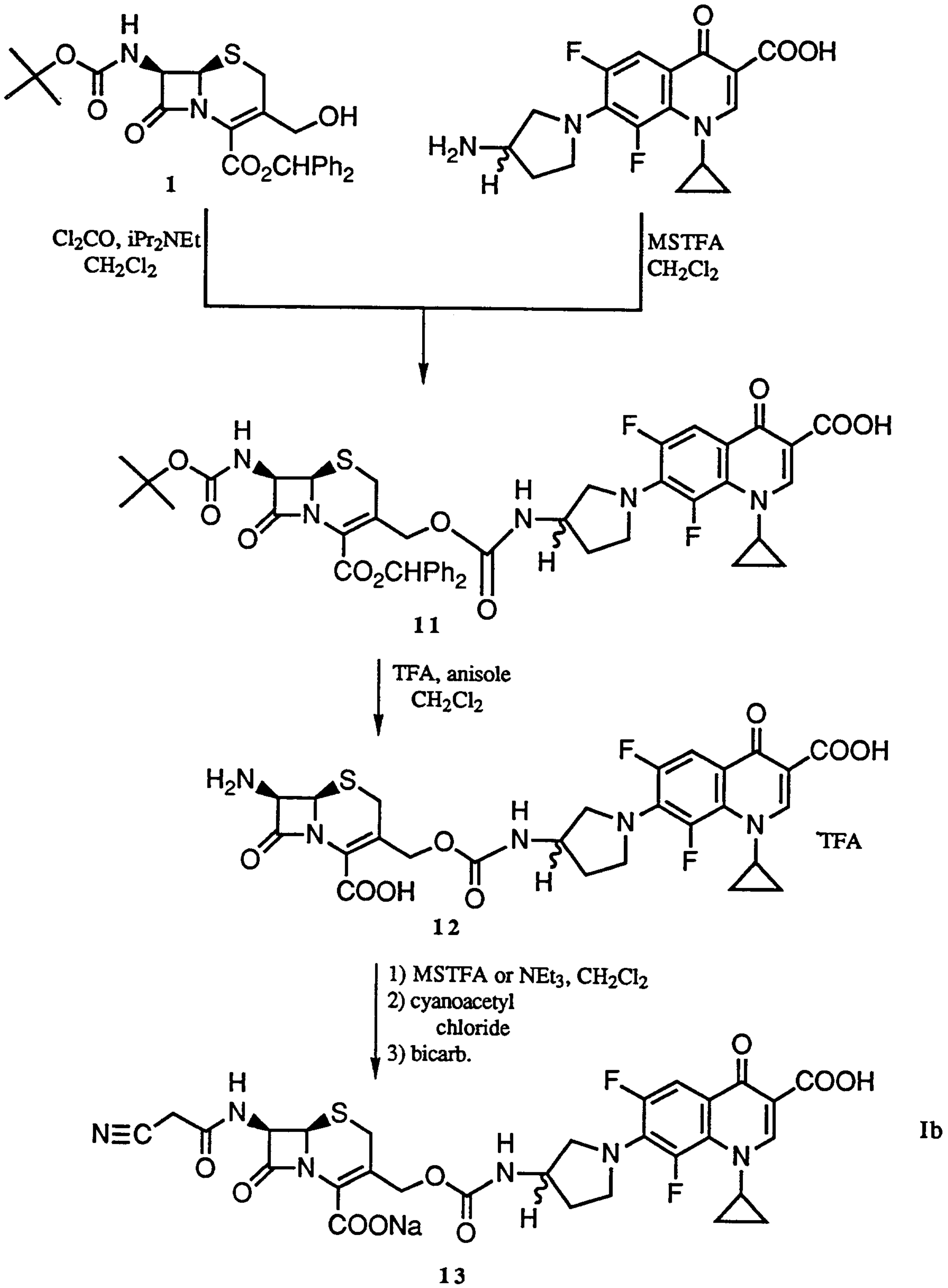


1

11

12

13

Ib

The starting materials in the reaction schemes are known and can be made by estabilished procedures; some are items of commerce, e.g., 7-aminocephalosphoranic acid. See, for example, Gordon, E.M. and Sykes, R.B. in "Chemistry and Biology of $\beta$-Lactam Antibiotics", Volume 1, Morin, R.B. and Gorman, M., Editors; Academic Press: New York, 1982; Chapter 3, and references therein, and Ponsford, R.J. et al. in "Recent Advances in the Chemistry of $\beta$-Lactam Antibiotics, Proceedings of the Third International

Symposium", Brown, A.G. and Roberts, S.M., Editors; Royal Society of Chemistry, Burlington House: London, 1985; Chapter 3, and references therein.

The compounds of the invention are shown by the following examples which are not meant to limit the invention. All temperatures are in degrees celsius unless otherwise stated. All methylene chloride referred to as "dry" in examples below indicated removal of residual water via distillation over calcium hydride and subsequent storage over 4 A sieves.

EXAMPLE 1

[6R,7R]-1-Cyclopropyl-7-[4[[[7-[[(1,1-dimethylethoxy)carbonyl]amino]-2-[(diphenylmethoxy)carbonyl]-8-oxo-5-thia-1-azabicyclo[4.2.0]oct-2-en-3-yl]methoxy]carbonyl]-1-piperazinyl]-6,8-difluoro-1,4-dihydro-4-oxo-3-quinoline-carboxylic acid (2)

Under anhydrous conditions and an argon atmosphere, 1-cyclopropyl-6,8-difluoro-1,4-dihydro-4-oxo-7-(1-piperazinyl)-3-quinolinecarboxylic acid (•4/5$H_2O$) (3.00 g, 8.24 mmol) was suspended in dry methylene chloride ($CH_2Cl_2$) (170 ml) at room temperature. N,O-Bis(trimethylsilyl)acetamide (BSA) (4.70 ml, 18.9 mmol) was added and the reaction became clear, homogeneous and nearly colorless. Stirring at room temperature was continued for 2 h. For the preparation of 1-cyclopropyl-6,8-difluoro-1,4-dihydro-4-oxo-7-(1-piperazinyl)-3-quinoline carboxylic acid, see United States Patent No. 4,556,658.

A dry three-necked round-bottom flask was equipped with two dropping funnels and purged with argon to provide a dry, oxygen-free atmosphere. In a separate dry vessel, (6R-trans)-3-hydroxymethyl-7-[[(1,1-dimethylethoxy)carbonyl]amino]-8-oxo-5-thia-1-azabicyclo[4.2.0]oct-2-ene-carboxylic acid diphenylmethyl ester **1**(7.40 g, 14.6 mmol) was dissolved in dry methylene chloride (150 ml) containing 4A molecular sieves. This solution was filtered to remove insoluble material and sieves and then placed in one dropping funnel. In a separate dry vessel, N,N-diisopropylethylamine ($iPr_2NEt$) (2.50 mi, 14.6 mmol) was dissolved in dry methylene chloride (150 ml) containing 4A molecular sieves and transferred to the remaining dropping funnel. In the bottom of the three-necked flask, with stirring, was placed phosgene ($Cl_2CO$) (7.60 ml of a 1.93 M solution in toluene, 14.6 mmol) in dry methylene chloride (150 ml). At this time the cephem solution and the amine solution were simultaneously added to the phosgene solution at approximately equal rates. The total addition time was approximately 10 min (~15 ml/min). After addition was completed, the reaction was stirred an additional 75 minutes at room temperature.

This crude chloroformylated cephem solution was transferred to a large dropping funnel and added to the silylated quinolone solution prepared as described above. This addition lasted approximately 12 minutes (~40 ml/min). After 45 min of stirring at room temperature, a solid was observed and thus an additional portion of BSA (1.0 ml, 4.0 mmol) was added. Stirring was continued for an additional 2 h and then a third portion of BSA (4.00 ml, 16.1 mmol) was added and the mixture stirred an additional 30 min. After this time, the solvents were removed via rotary evaporation. Ethyl acetate (500 ml) was added and the solution was filtered through a Celite pad to remove insoluble material. The pad was washed thoroughly with ethyl acetate (3 x 100 ml). The filtrate and washings were combined and placed in a separatory funnel. The organic extracts were washed with pH 4.0 buffer (2 x 150 ml). The combined aqueous washings were extracted with fresh ethyl acetate (1 x 100 ml). The combined organic extracts were washed with brine solution (1 x 100 ml), dried over magnesium sulfate and the solvents removed via rotary evaporation leaving a yellow solid.

This material was dissolved in a minimum volume of chloroform containing a trace of acetone and purified via silica gel column (25 cm x 2.5 cm O.D.) chromatography using a gradient elution. Elution with chloroform removed cephem by-products, and was followed by elution with 9:1 chloroform/methanol which caused a band of product to move down the column. Final elution with 6:1:6 chloroform/methanol/ECMA (EtOAc:100/$CHCl_3$:92/MeOH:7/HOAc:1) afforded pure product upon removal of solvents (6.4 g, 89%).

The (6R-trans)-3-hydroxymethyl-7-[[(1,1-dimethylethoxy)carbonyl]amino]-8-oxo-5-thia-1-azabicyclo-[4.2.0]oct-2-ene-carboxylic acid diphenylmethyl ester **1** was prepared according to the procedure set forth in European Patent Application No. 335 297, the pertinent parts of which are translated and presented below:

EXAMPLE 1A

Preparation of (6R-trans)-3-hydroxymethyl-7-[[(1,1-dimethyl-ethoxy)carbonyl]amino]-8-oxo-5-thia-1-azabicyclo[4.2.0]oct-2-ene-2-carboxylic acid

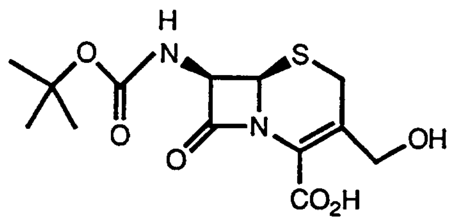

A solution of 19.2 g (0.48 mol) of sodium hydroxide in 240 ml of water was stirred and cooled to -5 °C in a bath of dry-ice and acetone. All at once, 54.4g (0.20 mol) of 7-aminocephalosporanic acid was added. The reaction temperature was controlled at -5 to 0 °C by raising and lowering the bath as necessary, until the initial heat of reaction was dissipated. The cold bath was then replaced with an ice bath and stirring was continued at 0-5 °C for the remainder of a total reaction time of 30 minutes. The pH was adjusted to 9-9.5 by addition of approximately 2 ml of 6 N hydrochloric acid. Dioxane (700 ml) was added followed by a solution of 87.5 g (0.40 mol) of di-tert-butyl dicarbonate in 200 ml of dioxane, added all at once. Sodium bicarbonate (33.6 g, 0.40 mol) was added, and the mixture stirred for 70 hours. Ethyl acetate (750 ml) was added, the layers were separated, and the organic phase was extracted with water (2 x 125 ml). The combined aqueous phase and extracts was washed with ethyl acetate (2 X 300 ml). Then the aqueous solution was layered with 750 ml of ethyl acetate, and 80 ml of 6 N HC1 was added to bring the pH to 2.5. A precipitate formed which was removed by filtration through a bed of Hyflow. The aqueous phase was separated and extracted with 300 ml of ethyl acetate. The organic extracts were combined, washed with 300 ml of water, dried ($Na_2SO_4$), and concentrated under reduced pressure to about 100 ml of volume. As the volume was reduced, solid material began to precipitate. The precipitate was filtered and washed with ether. The product was dried under reduced pressure; 24.1 g (36%) of the title compound was obtained. This was used directly in the next Example.

EXAMPLE 1B

Preparation of (6R-trans)-3-hydroxymethyl-7-[[(1,1-dimethyl-ethoxy)carbonyl]amino]-8-oxo-5-thia-1-azabicyclo[4.2.0]oct-2-ene-carboxylic acid diphenylmethyl ester (1)

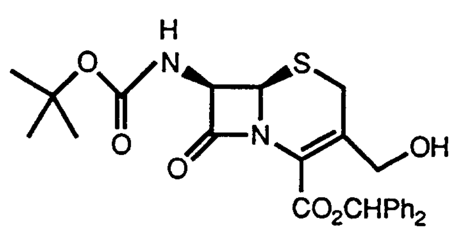

A suspension of 44.67 g (0.135 mol) of the product (hydroxy acid), from Example 1A, in 270 ml of dry THF, was stirred mechanically. A solution of 32.51 g (0.167 mol) of diphenyldiazomethane in 390 ml of hexanes was added, and the mixture was stirred vigorously for 20 hours. The precipitate was filtered, washed with hexanes, and dried under reduced pressure to obtain 45.74 g (68.2%) of the title compound: 1H NMR ($Me_2SO$-$d_6$) δ1.41 (s, 9H), 3.59 (s, 2H), 4.22 (t, J=6Hz, 2H), 5.08 (d, J=5Hz), 5.51 (dd, J=5 and 8 Hz, 1H), 6.88 (s, 1H), 7.26-7.51 (m, 10H), 8.03 (d, J=8Hz, 1H).

EXAMPLE 2

[6R,7R]-7-Amino-3-[[[[4-(3-carboxy-1-cyclopropyl-6,8-difluoro-1,4-dihydro-4-oxo-7-quinolinyl)-1-piperazinyl]-carbonyl]oxy]-methyl]-8-oxo-5-thia-1-azabicyclo[4.2.0]oct-2-ene-2-carboxylic acid trifluoroacetate salt (3)

Product **2** from Example 1(6.40 g, 7.34 mmol) was dissolved in dry methylene chloride under an argon atmosphere and cooled to ~0 ° C in an ice bath. Anisole (1.76 ml, 16.1 mmol) was added followed by the addition of trifluoroacetic acid (40 ml). The reaction was stirred at ~0 ° C for 30 min and then the cooling bath was removed and the reaction allowed to warm and stir at room temperature for 4 h. After this time, the solvents were removed by rotary evaporation leaving a viscous oil. Fresh chloroform (~100ml) was added and then removed by rotary evaporation and the resultant oil was triturated with ethyl ether to obtain a tan solid which was collected by filtration and washed thoroughly with fresh ether (4x). The solid **3** was dried on a vacuum pump (5.4g, ~100%).

EXAMPLE 3

[6R,7R]-3-[[[[4-(3-Carboxy-1-cyclopropyl-6,8-difluoro-1,4-dihydro-4-oxo-7-quinolinyl)-1-piperazinyl]carbonyl]-oxy]-methyl]-8-oxo-7-[(2-thienylacetyl)amino]-5-thia-1-azabicyclo-[4.2.0]oct-2-ene-2-carboxylic acid (4)

Product **3** from Example 2 (5.4 g, 7.34 mmol) was suspended in dry methylene chloride (270 ml) at room temperature under an argon atmosphere. N-Methyl-N-(trimethylsilyl)trifluoroacetamide (MSTFA) (7.8 ml, 42 mmol) was added which resulted in a clear homogeneous golden solution. After 40 minutes, 2-thiopheneacetyl chloride (1.35 ml, 11.0 mmol) was added in a dropwise manner. A turbid tan-pale brown solution resulted which was stirred for 2 h. The reaction was concentrated via rotary evaporation to approximately 30 ml volume and then triturated with ethyl ether to obtain a tan solid which was collected via filtration and thoroughly washed with fresh ether (5x). The product **4** was further dried on a vacuum pump (5.0 g, 93%). This product was obtained in microcrystalline form as follows: product **4** (250 mg) was suspended/dissolved in acetone (5 ml reagent grade). 1.0 N HC1 solution (2 ml) was added and the mixture cooled in an ice-acetone bath (~3 ° C). The resultant pale yellow solid was filtered and washed with cold (~3 ° C) water and then with cold acetone. The monosodium carboxylate salt of product **4** was obtained in high purity upon reverse-phase column chromatography at neutral or slightly basic pH (sodium bicarbonate or pH 8.2 buffer/aqueous acetronitrile).

EXAMPLE 4

[6R-[3(S*),6 alpha, 7 beta]]-1-Cyclopropyl-7-[3-[[[[[7-[[(1,1-dimethylethoxy)carbonyl]amino]-2-[-(diphenylmethoxy)-carbonyl]-8-oxo-5-thia-1-azabicyclo[4.2.0]oct-2-en-3-yl]methyl]oxy]carbonyl]amino]-1-pyrrolidinyl]-6,8-difluoro-1,4-dihydro-4-oxo-3-quinoline carboxylic acid (5)

In a dry vessel at room temperature (S)-7-(3-amino-1-pyrrolidinyl)-1-cyclopropyl-6,8-difluoro-1,4-dihydro-4-oxo-3-quinolinecarboxyiic acid monohydrochloride salt (3.39 g, 8.79 mmol) was suspended in 250 ml of dry methylene chloride. N-Methyl-N-(trimethylsilyl)trifluoroacetamide (MSTFA) (8.5 ml, 45.8 mmol) was added and the resultant clear homogeneous solution was stirred for 30 min.

Under an argon atmosphere, a solution of (6R-trans)-3-hydroxymethyl-7-[[(1,1-dimethylethoxy)carbonyl]-amino]-8-oxo-5-thia-1-azabicyclo[4.2.0]-oct-2-ene-carboxylic acid diphenylmethyl ester **1** (7.53 g, 15.2 mmol) in 250 ml of dry methylene chloride was stirred over 4 A molecular sieves for 30 min and then transferred to a dropping fUnnel by means of a cannula. This solution was added simultaneously with a solution of N,N-diisopropylethylamine ($iPr_2NEt$) (2.6 ml, 15.2 mmol) in 250 ml of dry methylene chloride to cold (0 ° -5 ° C) solution of phosgene (7.9 ml of 1.93M solution in toluene, 15.2 mmol) in 250 ml of dry methylene chloride. The simultaneous additions were complete in 12 min, after which time stirring was continued at 0 ° -5 ° C for an additional 10 min. The cooling bath was removed and the reaction mixture was stirred at ambient temperature for 45 min. After this time, the reaction was concentrated under high vacuum for 20 min, allowing reaction mixture to cool as the solvent was removed. This cooled solution of the intermediate cephem chloroformate was then added (using a cannula) to the solution of silylated quinolone prepared as described above and cooled to ~0 ° -5 ° C. The addition of the cephem chloroformate solution took 11 min. The reaction mixture was then stirred for an additional 10 min at ~0 ° -5 ° C after which time the bath was removed and the mixture stirred at ambient temperature for 3 h. The reaction mixture was treated with pH 4 buffer solution (45 ml), stirring vigorously for 15 min. The insoluble material was removed by

filtration and the filtrate was washed with 10:1 pH 4 buffer/brine (3 X 125 ml) and then with brine (1 x 125 ml). The organic phase was dried ($Na_2SO_4$) and concentrated to a small volume by rotary evaporation. Diethyl ether was added to precipitate the product and the mixture was chilled (-20 °C) overnight. The solid was collected by filtration, yielding 4.38 g (57.1% of theory) of the title compound **5**.

EXAMPLE 5

[6R-[3(S*), 6 alpha, 7 beta]]-7-Amino-3-[[[[[1-(3-carboxy-1-cyclopropyl-6,8-difluoro-1,4-dihydro-4-oxo-7-quinolinyl)-3-pyrrolidinyl]amino]carbonyl]oxy]methyl]-8-oxo-5-thia-1-azabicyclo[4.2.0]oct-2-ene-2-carboxylic acid trifluoroacetate salt (6)

A solution of product **5** from Example 4 (8.40 g, 9.63 mmol) and anisole (2.5 ml, 23.0 mmol) in methylene chloride (160 ml) was cooled to 0 °C, under an argon atmosphere. Cold (0 °-5 °C) trifluoroacetic acid (TFA) (160 ml) was added dropwise to the solution over 24 min. The reaction was stirred at 0 °-5 °C for 3 h and 45 min and then the bath was removed and stirring continued at ambient temperature for 15 min. The reaction was concentrated by rotary evaporation to a deep red oily gum residue. Methylene chloride was added and the mixture was concentrated again. Ethyl ether was added to the oily gum residue and the mixture was stirred, and the walls of the flask scraped down with a spatula, until the deep red oily gum residue had completely solidified to a yellow solid. The solid was filtered, washed with ether, and dried under high vacuum to obtain 7.05 g (100%) of the title compound **6**.

EXAMPLE 6

[6R-[3(S*), 6 alpha, 7 beta]]-3-[[[[[1-(3-Carboxy-1-cyclopropyl-6,8-difluoro-1,4-dihydro-4-oxo-7-quinolinyl-3-pyrrolidinyl]-amino]carbonyl]oxy]methyl]-7-[(cyanoacetyl)amino]-8-oxo-5-thia 1-azabicyclo[4.2.0]oct-2-ene-2-carboxylic acid monosodium salt (7)

Cyanoacetyl chloride was prepared by adding oxalyl chloride (1.0 ml, 11.5 mmol) to suspension of cyanoacetic acid (0.98 g, 11.5 mmol) and dimethylformamide (15 µL) in methylene chloride (25 ml) at 0 °C, and under an argon atmosphere. The mixture was stirred for 5 min and then the cooling bath was removed and stirring continued at ambient temperature for 2 h. The clear solution was diluted with 10 ml of methylene chloride and concentrated under high vacuum to less than the original volume. Methylene chloride (40 ml) was added to the residual solution making the volume of the cyanoacetyl chloride solution approximately 60 ml.

In a separate vessel, product **6** from Example 5 (4.5 g, 6.25 mmol) was suspended in dry methylene chloride (65 ml) and cooled to 0 °C. Triethylamine (3.9 ml, 28.0 mmol) was added to the suspension dropwise until all of the solid had reacted and was solubilized. (As an alternate method, N-methyl-N-(trimethylsilyl)trifluoroacetamide (MSTFA) may be used in place of triethylamine. When MSTFA is used, the acidification with HCl, as described below, is not needed). The cyanoacetyl chloride solution was then added to the cephem over an 11 min period. The mixture was stirred at 0 °-5 °C for 80 minutes. The reaction mixture was concentrated and the resultant oily gum residue was solublised by the addition of a mixture of pH 7.8 buffer and saturated aqueous sodium bicarbonate, keeping the pH of the mixture in the range of 7.8-8.1. This turbid solution was acidified to pH 4 with 2 N HCl. The solid was collected by filtration and washed several times with water to insure complete removal of triethylamine hydrochloride. This solid was then dissolved in the buffer-bicarbonate mixture and acetonitrile was added until this solution contained ~5% acetonitrile by volume. This turbid solution was purified via $C_{18}$ silica reverse phase column chromatography (column size: 5.4 cm O.D. x 15 cm). The product was eluted from the column with a gradient of acetonitrile/pH 7.8 buffer (8% to 20% acetonitrile). Pure product-containing fractions were combined and freeze-dried. (Alternately, this freeze dry step can be eliminated and the product-containing fractions desalted after removal of acetonitrile.) The freeze-dried material was dissolved and "desalted" on a short $C_{18}$ silica gel column (column size: 8.4 cm O.D. x 5 cm). The column was washed with 3 L (12-14 column volumes) of water and then the product was eluted with 3:1 water/acetonitrile. The pure product-containing fractions were freeze-dried giving 1.75 g (41%) ofthe title compound **7**. Additional product (0.80 g, 18%) was obtained by ffirther purification of the less pure fractions (59% total yield).

EXAMPLE 7

[6R-[3(R*), 6 alpha, 7 beta]]-1-Cyclopropyi-7-[3-[[[[[7-[[(1,1-dimethylethoxy)carbonyl]amino]-2-[-(diphenylmethoxy)carbonyl]-8-oxo-5-thia-1-azabicyclo[4.2.0]oct-2-en-3-yl]methyl]oxy]carbonyl]amino]-1-pyrrolidinyl]-6,8-difluoro-1,4-dihydro-4-oxo-3-quinoline carboxylic acid (8)

The title compound **8** was prepared from (R)-7-(3-amino-1-pyrrolidinyl)-1-cyclopropyl-6,8-difluoro-1,4-dihydro-4-oxo-3-quinolinecarboxylic acid using the procedure described above in Example 4.

EXAMPLE 8

[6R-[3(R*), 6 alpha, 7 beta]]-7-Amino-3-[[[[[1-(3-carboxy-1-cyclopropyl-6,8-difluoro-1,4-dihydro-4-oxo-7-quinolinyl)-3-pyrrolidinyl]amino]carbonyl]oxy]methyl]-8-oxo-5-thia-1-azabicyclo[4.2.0]oct-2-ene-2-carboxylic acid trifluoroacetate salt (9)

The title compound **9** was prepared from **8** using the procedure described above in Example 5.

EXAMPLE 9

[6R-[3(R*), 6 alpha, 7 beta]]-3-[[[[[1-(3-Carboxy-1-cyclopropyl-6,8-difluoro-1,4-dihydro-4-oxo-7-quinolinyl-3-pyrrolidinyi]amino]carbonyl]oxy]methyL]-7-[(cyanoacetyl)amino]-8-oxo-5-thia-1-azabicyclo[4.2.0]oct-2-ene-2-carboxylic acid monosodium salt (10)

The title compound **10** was prepared from **9** using the procedure described above in Example 6.

EXAMPLE 10

(6R,7R]-1-Cyclopropyl-7-[3-[[[[[7-[[(1,1-dimethylethoxy)-carbonyl]amino]-2-[(diphenylmethoxy)carhonyl]-8-oxo-5-thia-1-azabicyclo[4.2.0]oct-2-en-3-yl]methyl]oxy]carbonyl]amino]-1-[R,S]-pyrrolidinyl]-6,8-difluoro-1,4-dihydro-4-oxo-3-quinoline carboxylic acid (11)

The title compound **11** was prepared from racemic 7-(3-amino-1-pyrrolidinyl)-1-cyclopropyl-6,8-difluoro-1,4-dihydro-4-oxo-3-quinolinecarboxylic acid using the procedure described above in Example 4.

EXAMPLE 11

[6R,7R]-7-Amino-3-[[[[[1-(3-carboxy-1-cyclopropyl-6,8-difluoro-1,4-dihydro-4-oxo-7-quinolinyl)-3-[R,S]-pyrrolidinyl]-amino]carbonyl]oxy]methyl]-8-oxo-5-thia-1-azabicyclo[4.2.0]oct-2-ene-2-carboxylic acid trifluoroacetate salt (12)

The title compound **12** was prepared from **11** using the procedure described above in Example 5.

EXAMPLE 12

[6R,7R]-3-[[[[[1-(3-Carboxy-1-cyclopropyl-6,8-difluoro-1,4-dihydro-4-oxo-7-quinolinyl-3-[R,S]-pyrrolidinyl]-amino]carbonyl]oxy]methyl]-7-[(cyanoacetyl)amino]-8-oxo-5-thia-1-azabicyclo[4.2.0]oct-2-ene-2-carboxyiic acid monosodium salt (13)

The title compound **13** was prepared from **12** using the procedure described above in Example 6.

The quinolones used in Examples 4,7 and 10 were prepared as follows:

(S)-7-(3-amino-1-pyrrolidinyl)-1-cyclopropyl-6,8-difluoro-1,4-dihydro-4-oxo-3-quinoline carboxylic acid was prepared by reacting 1-cyclopropyl-6,7,8-trifluoro-1,4-dihydro-4-oxo-3-quinolone carboxylic acid with (S)-3-(acetylamino)-1-pyrrolidine by heating in pyridine and treating with HCl. The 1-cyclopropyl-6,7,8-trifluoro-1,4-dihydro-4-oxo-3-quinoline carboxylic acid was prepared as set forth in United States Patent No. 4,655,079.

(R)-7-(3-amino-1-pyrrolidinyl)-1-cyclopropyl-6,8-difluoro-1,4-dihydro-4-oxo-3-quinoline carboxylic acid was prepared by reacting 1-cyclopropyl-6,7,8-trifluoro-1,4-dihydro-4-oxo-3-quinoline carboxylic acid with (R)-3-(acetylamino)-1-pyrrolidine by heating in pyridine and treating with HCl.

Racemic 7-(3-amino-1-pyrrolidinyl)-1-cyclopropyl-6,8-difluoro-1,4-dihydroxy-4-oxo-3-quinoline carboxylic acid was prepared by reacting 1-cyclopropyl-6,7,8-trifluoro-1,4-dihydro-4-oxo-3-quinoline carboxylic acid, 3-[(tert-butoxycarbonyl)-amino]pyrrolidine, and triethylamine together in acetonitrile to form 7-[3-(tert-butoxycarbonyl)amino]-1-pyrrolidinyl-]-1-cyclopropyl-6,8-difluoro-1,4-dihydro-4-oxo-3-quinoline carboxylic acid which was then suspended in 6M HCl and acetic acid and heated to 60 °C for 4 h and then stirred at room temperature (eg. 21 °C) overnight. The solution was filtered and concentrated. The residue was triturated with EtOH:ether (1:1) and the solids were washed with ether to give the above mentioned quinolone.

## Claims

**1.** Cephalosporin derivatives of the general formula

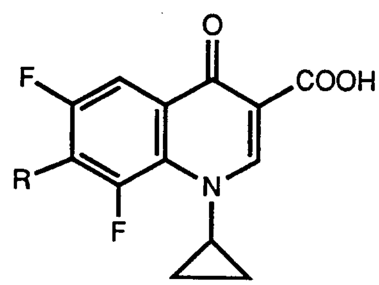

I

in which R represents one of the groups

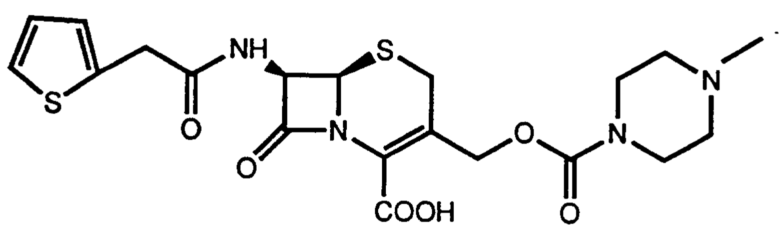

(a) and

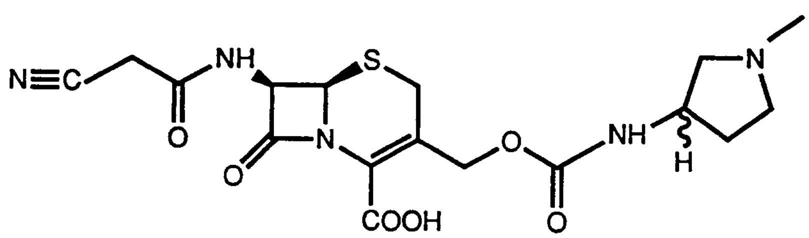

(b)

and the corresponding readily hydrolyzable, pharmaceutically acceptable esters and the pharmaceutically acceptable salts of these compounds and bydrates of compounds of formula I and of their esters and salts.

**2.** A compound according to claim 1 of the formula

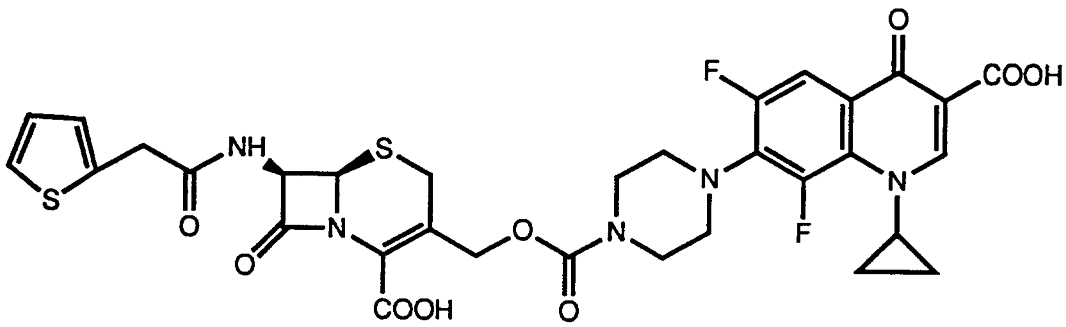

Ia

and the corresponding readily hydrolyzable, pharmaceutically acceptable esters and the pharmaceutically acceptable salts of this compound and hydrates of the compound of formula Ia and of its esters and salts.

3. A compound according to claim 1 of the formula

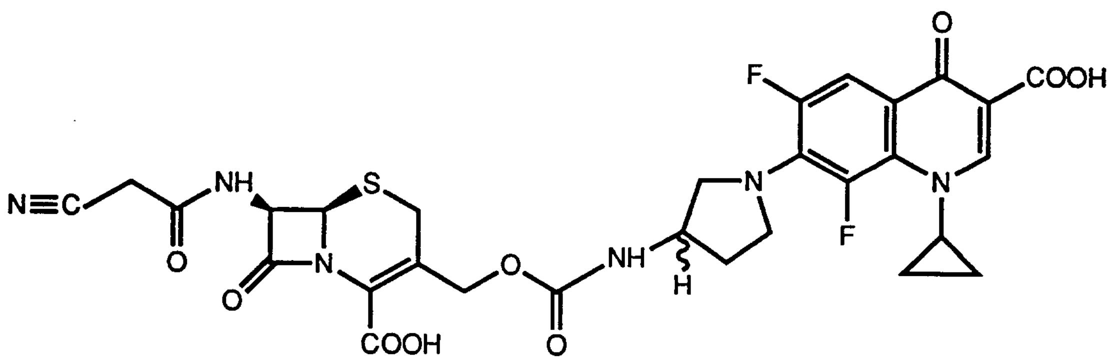

Ib

and the corresponding readily hydrolyzable, pharmaceutically acceptable esters and the pharmaceutically acceptable salts of this compound and hydrates of the compound of formula Ib and of its esters and salts.

4. A compound according to claim 1 having the formula

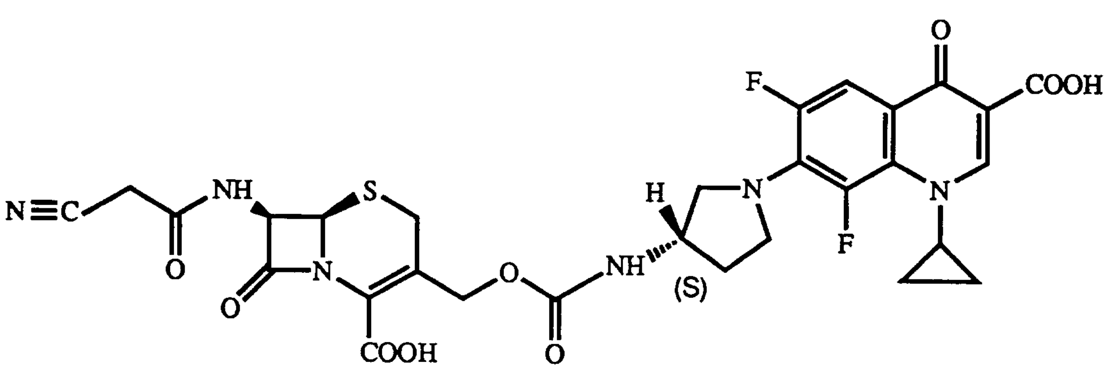

Iba

and the corresponding readily hydrolyzable, pharmaceutically acceptable esters and the pharmaceutically acceptable salts of this compound and hydrates of the compound of formula Iba and of its esters and salts.

5. A compound according to claim 4 of the formula

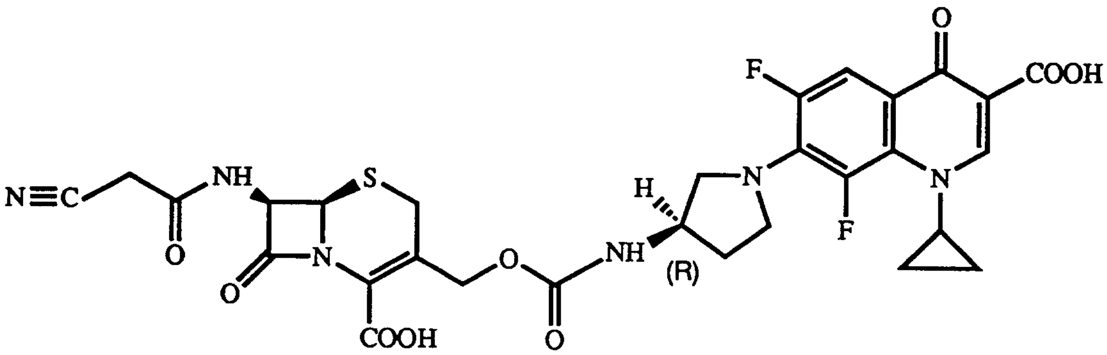

Ibb

and the corresponding readily hydrolyzable, pharmaceutically acceptable esters and the pharmaceutically acceptable salts of this compound and hydrates of the compound of formula Ibb and of its esters and salts.

**6.** Compounds according to any one of claims 1-5 for use as therapeutically active substances.

**7.** Compounds according to any one of claims 1-5 for use as antibiotically active substances.

**8.** A process for the manufacture of the compounds in accordance with any one of claims 1-5, which process comprises
a) acylating a compound of the general formula

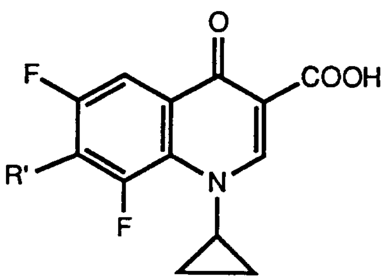

II

in which R' is a group corresponding to R carrying an unsubstituted amino group in position 7 of the cephalosporin nucleus,
or an acid addition salt thereof, or
b) for the manufacture of a readily hydrolyzable, pharmaceutically acceptable ester of a compound of formula I subjecting a carboxylic acid of formula I to a corresponding esterification, or
c) for the manufacture of pharmaceutically acceptable salts and hydrates of a compound of formula I and of hydrates of said salts converting a compound of formula I into a pharmaceutically acceptable salt or hydrate or into a hydrate of this salt.

**9.** A medicament containing a compound according to any one of claims 1-5 and a therapeutically inert carrier.

**10.** An antibiotically-active medicament containing a compound according to any one of claims 1-5 and a therapeutically inert carrier.

**11.** The use of compounds according to any one of claims 1-5 in the control or prevention of illnesses.

**12.** The use of compounds according to any one of claims 1-5 in the control or prevention of infectious diseases.

**13.** The use of compounds according to any one of claims 1-5 in the manufacture of antibiotically active medicaments.